# EUROPEAN PATENT APPLICATION

(11) **EP 3 305 780 A1**
(43) Date of publication of application: **11.04.2018**
(21) Application number: 16192345.3
(22) Date of filing: 05.10.2016
(51) Int. Cl.: C07D 403/12, C07D 209/30

(54) **METHOD FOR THE PREPARATION OF H-ARG(MIS)-OH**

(71) Applicant: Lonza Ltd, 3930 Visp (CH)
(72) Inventor: The designation of the inventor has not yet been filed

(57) **Abstract**

The invention discloses a method for the preparation of N-omega-(1,2-dimethylindole-3-sulfonyl)-L-arginine and its derivatives using L-omithine.

## Description

The invention discloses a method for the preparation of N-omega-(1,2-dimethylindole-3-sulfonyl)-L-arginine and its derivatives using L-ornithine.

### BACKGROUND OF THE INVENTION

WO 2009/135645 A1 discloses a new protecting group for Arg that is derived from indole-3-sulfonyl chloride. It can advantageously be used in peptides synthesis for protecting Arg or other amino acids and has better performance compared to hitherto known protecting groups such as Pbf, this better performance is exemplified in WO'645.

The method for preparation for Arg protected with MIS in form of H-Arg(MIS)-OH, that is disclosed in WO'645 in examples 1 to 4, has the sequence as given in Formula Scheme 1:

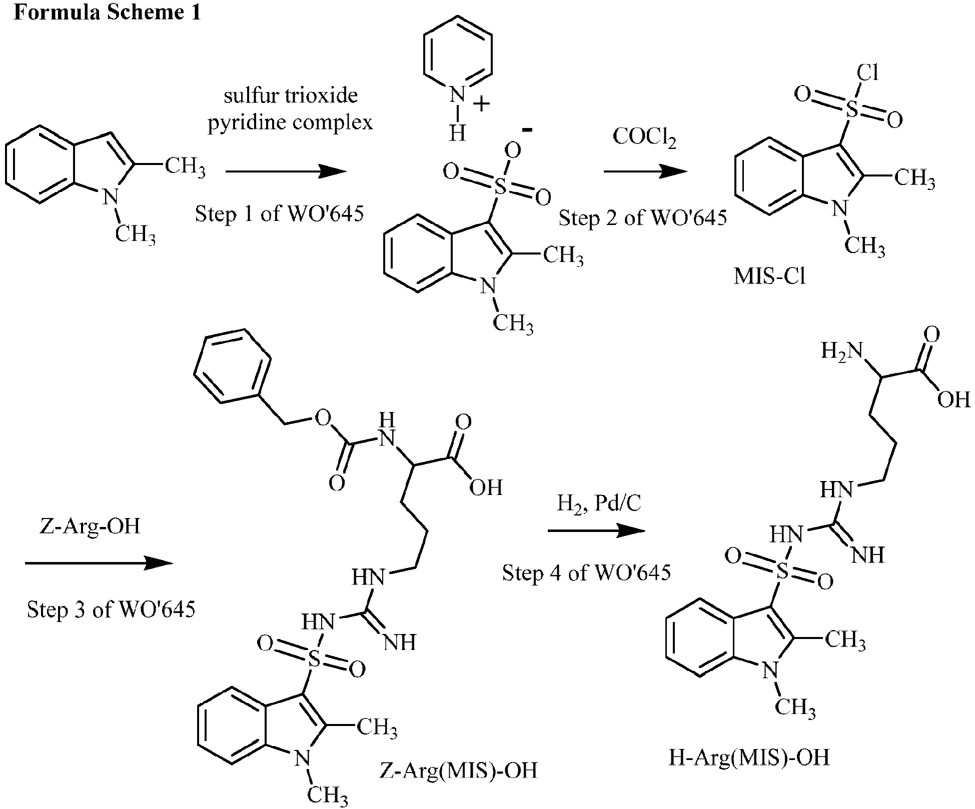

The yield of the steps is given in WO'645 with 96% for Step 1, 78% for Step 2, 18% for Step 3 and 98% for Step 4. Especially Step 3 of WO'645 shows a low yield.

There was a need for a method for preparation of H-Arg(MIS)-OH with a better yield than disclosed in WO'645.
The method of present invention shows higher yield.

The following abbreviations and meanings are used, if not stated otherwise:
Compound of formula (1) 1,2-dimethyl-1H-indol, 1,2-dimethylindol
Compound of formula (2) MIS-OH.Py, Pyridinium 1,2-dimethyl-1H-indole-3-sulfonate
Compound of formula (3) MIS-Cl, 1,2-Dimethyl-1H-indole-3-sulfonyl chloride
Compound of formula (5) L-Arg(MIS)-OH
Compound of formula (6) Fmoc-Arg(MIS)-OH
   Compound of formula (PYCAAM) Pyrazole-1-carboxamidine
   Compound of formula (SULPYR) sulphur trioxide pyridine complex
      DMF Dimethylformamide
      DIPEA Diisopropylethylamin
      DMAA Dimethylacetamide
      DMPU 1,3-Dimethyl-3,4,5,6-tetrahydro-2(1H)-pyrimidinone
      DMSO Dimethyl sulfoxide
      EDTA Ethylenediaminetetraacetic acid
      Fmoc fluoren-9-ylmethoxycarbonyl
      Fmoc-OSu Fmoc N-hydroxysuccinimide ester, compound of formula (Fmoc-OSu)
      halogen F, Cl, Br or I, preferably F, Cl or Br, more preferably F or Cl; even more preferably Cl
      L-Orn.HCl L-ornithine hydro chloride
      MTBE Methyl tert-butyl ether
      MIS 1,2-dimethylindol-3-sulfonyl
      NMP N-Methyl-2-pyrrolidone
      Pbf 2,2,4,6,7-pentamethyldihydrobenzofuran-5-sulfonyl
      WO'645 WO 2009/135645 A1
      Z benzyloxycarbonyl

### SUMMARY OF THE INVENTION

Subject of the invention is a method for the preparation of compound of formula (V) 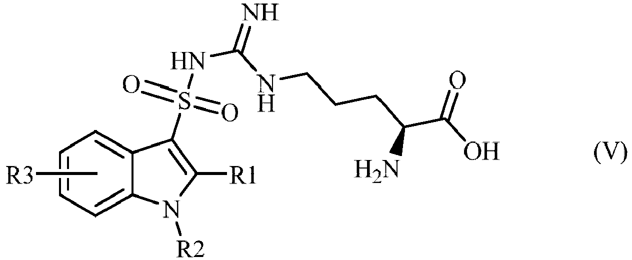 by a reaction REAC4, wherein compound of formula (IV) 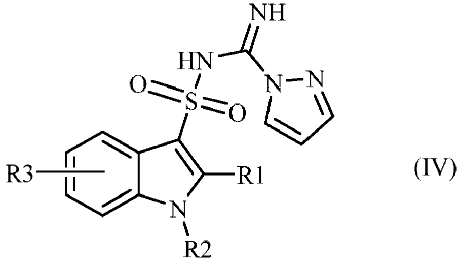 is reacted with L-ornithine;
R1 is H, C₁₋₆ alkyl, C₁₋₆ alkoxy or C₁₋₆ alkylthio;
R2 is C₁₋₆ alkyl, C₁₋₆ alkoxy or C₁₋₆ alkylthio;
or R1 and R2 together form a moiety of formula -(CH₂)n-,
n 3, 4 or 5;
R3 is H, halogen, C₁₋₆ alkyl, C₁₋₆ alkoxy, C₁₋₆ alkylthio, phenyl or benzyl.

### DETAILED DESCRIPTION OF THE INVENTION

Preferably,
R1 is C₁₋₄ alkyl, C₁₋₄ alkoxy or C₁₋₄ alkylthio;
R2 is C₁₋₄ alkyl, C₁₋₄ alkoxy or C₁₋₄ alkylthio;
R3 is H, halogen, C₁₋₄ alkyl, C₁₋₄ alkoxy, C₁₋₄ alkylthio, phenyl or benzyl;
more preferably,
R1 is C₁₋₄ alkyl, C₁₋₄ alkoxy or C₁₋₄ alkylthio;
R2 is C₁₋₄ alkyl, C₁₋₄ alkoxy or C₁₋₄ alkylthio;
R3 is H, halogen, C₁₋₄ alkyl, C₁₋₄ alkoxy or C₁₋₄ alkylthio;
even more preferably,
R1 is C₁₋₄ alkyl or C₁₋₄ alkoxy;
R2 is C₁₋₄ alkyl or C₁₋₄ alkoxy;
R3 is H, halogen, C₁₋₄ alkyl or C₁₋₄ alkoxy;
especially,
R1 is C₁₋₄ alkyl or C₁₋₄ alkoxy;
R2 is C₁₋₄ alkyl or C₁₋₄ alkoxy;
R3 is H or halogen;
more especially,
R1 is C₁₋₂ alkyl or C₁₋₂ alkoxy;
R2 is C₁₋₂ alkyl or C₁₋₂ alkoxy;
R3 is H or halogen;
even more especially,
R1 is C₁₋₂ alkyl;
R2 is C₁₋₂ alkyl;
R3 is H or halogen.

Preferably, REAC4 is done at a pH of from 9 to 12, more preferably of from 10 to 12, more preferably from 10.5 to 11.5.
Preferably, the pH is adjusted by the use of LiOH, NaOH or KOH, more preferably LiOH.

Preferably, L-ornithine is used in form of its hydrochloride.
Preferably, the molar amount of L-ornithine is from 1 to 5 times, more preferably from 1 to 3 times, even more preferably from 1 to 2.5 times, of the molar amount of compound of formula (IV).

Preferably, the reaction temperature TEMP4 of REAC4 is from 0 to 100°C, more preferably from 10 to 80°C
Preferably, the pressure PRESS4 of REAC4 is adjusted according to the vapor pressure of the reaction mixture at the chosen TEMP4 of REAC4; but PRESS4 can also be adjusted to a higher pressure than the vapor pressure of the reaction mixture at the chosen TEMP4. A PRESS4 higher than the vapor pressure of the reaction mixture at the chosen TEMP4 can be adjusted for example by applying inert gas such a nitrogen or argon to the reaction vessel;
more preferably, REAC4 is done at a pressure from atmospheric pressure to 20 bar, more preferably from atmospheric pressure to 10 bar, even more preferably from atmospheric pressure to 5 bar, REAC4 is done at atmospheric pressure.

Preferably, the reaction time TIME4 of REAC4 is from 6 h to 48 h, more preferably from 12h to 36 h, and even more preferably from 12 h to 24 h.

REAC4 can be performed under an atmosphere of air or under an atmosphere of an inert gas, such as nitrogen or such as a noble gas, such as argon.

REAC4 can be done in a solvent SOLV4, SOLV4 is preferably water, acetonitrile, MeOH, DMF, DMSO, tetramethylurea, DMPU, or a mixture thereof;
more preferably water, acetonitrile, or a mixture thereof.
Preferably, REAC4 is done in SOLV4.

After REAC4, compound of formula (V) can be isolated and purified by conventional methods, which are known to those skilled in the art. These conventional methods include extraction, chromatography, or any combination of these methods of purification.

Preferably, after REAC4 compound of formula (V) is precipitated by the addition of a salt of Cu(II), preferably the salt of Cu(II) is CuCl₂, CuSO₄, or Cu(NO₃)₂; more preferably CuCl₂.
In case of said precipitation a salt of Cu(II), the Cu(II) is preferably removed by known methods such as filtration, washing of the filter cake with water or EtOH, or extraction with a substance that forms an salt with Cu(II), such as EDTA, or a combination of these methods. More preferably, after REAC4 compound of formula (V) is isolated by precipitation by the addition of a salt of Cu(II), filtration of the precipitate, washing of the filter cake formed by the filtration, preferably with water and EtOH, mixing the filter cake with water and using EDTA for removal of residual Cu(II) ions.
Preferably, the molar amount of the Cu(II) salt is from 0.1 to 10 times, more preferably of from 0.5 to 5 times, of the molar amount of compound of formula (IV).

Preferably, compound of formula (IV) is prepared by a reaction REAC3 of compound of formula (III) with compound of formula (PYCAAM); 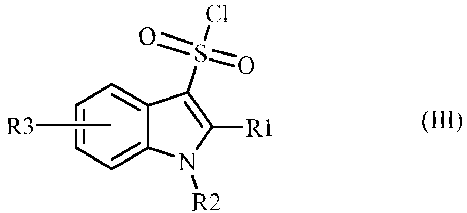 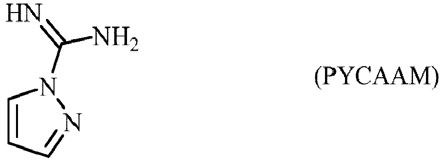 with R1, R2 and R3 as defined herein, also with all their embodiments.

Preferably, compound of formula (PYCAAM) is used in REAC3 in form of its hydrochloride.

Preferably, the molar amount of compound of formula (PYCAAM) is from 1 to 5 times, more preferably from 1 to 3 times, even more preferably from 1 to 2 times, of the molar amount of compound of formula (III).

Preferably, the reaction temperature TEMP3 of REAC3 is from 0 to 100°C, more preferably from 0 to 80°C, even more preferably from 10 to 60°C, especially from 10 to 40°C, more especially from 15 to 30°C.
Preferably, the pressure PRESS3 of REAC3 is adjusted according to the vapor pressure of the reaction mixture at the chosen TEMP3 of REAC4; but PRESS3 can also be adjusted to a higher pressure than the vapor pressure of the reaction mixture at the chosen TEMP3. A PRESS3 higher than the vapor pressure of the reaction mixture at the chosen TEMP3 can be adjusted for example by applying inert gas such a nitrogen or argon to the reaction vessel;
more preferably, REAC3 is done at a pressure from atmospheric pressure to 20 bar, more preferably from atmospheric pressure to 10 bar, even more preferably from atmospheric pressure to 5 bar, REAC3 is done at atmospheric pressure.
Preferably, the reaction time TIME3 of REAC3 is from 6 h to 48 h, more preferably from 12h to 36 h, and even more preferably from 12 h to 24 h.

REAC3 can be performed under an atmosphere of air or under an atmosphere of an inert gas, such as nitrogen or such as a noble gas, such as argon.

REAC3 can be done in a solvent SOLV3, SOLV3 is preferably C₁₋₄ alkyl nitrile, dichlorethane, methylene chloride, triethyl amine, diisopropyl amine, MTBE, DMSO, DMF, DMAA, NMP, or a mixture thereof;
more preferably acetonitrile.
Preferably, REAC3 is done in SOLV3.

After REAC3, compound of formula (IV) can be isolated and purified by conventional methods, which are known to those skilled in the art. These conventional methods include extraction, crystallization, chromatography, or any combination of these methods of purification.

Preferably, compound of formula (III) is prepared by a reaction REAC2 of compound of formula (II) with a halogenating agent HALAG2; 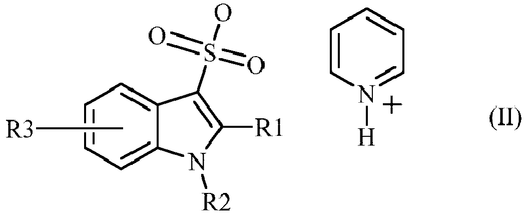 with R1, R2 and R3 as defined herein, also with all their embodiments.

Preferably, HALAG2 is selected from the group consisting of oxalyl chloride, thionylchloride, phosgene, diphosgene, triphosgene, POCl₃, PCl₅, Cl₂ in the presence of triphenylphoshine, cyanuric chloride, and methansulfonic acid chloride;
more preferably, HALAG2 is oxalyl chloride.
Preferably, the molar amount of HALAG2 is from 1 to 5 times, more preferably from 1 to 3 times, even more preferably from 1 to 2 times, of the molar amount of compound of formula (II).

REAC2 can be done in the presence of a base BAS2; preferably, BAS2 is DMF, picoline, chinoline, pyrimidine, N-C₁₋₂ alkyl imidazole;
more preferably, BAS2 is DMF.
Preferably, the molar amount of BAS2 is from 0.01 to 1 times, more preferably from 0.05 to 0.5 times, even more preferably from 0.05 to 0.3 times, of the molar amount of compound of formula (II).

Preferably, the reaction temperature TEMP2 of REAC2 is from -10 to 50°C, more preferably from -10 to 20°C, even more preferably from -10 to 10°C.
Preferably, the pressure PRESS2 of REAC2 is adjusted according to the vapor pressure of the reaction mixture at the chosen TEMP2 of REAC2; but PRESS2 can also be adjusted to a higher pressure than the vapor pressure of the reaction mixture at the chosen TEMP2. A PRESS2 higher than the vapor pressure of the reaction mixture at the chosen TEMP2 can be adjusted for example by applying inert gas such a nitrogen or argon to the reaction vessel;
more preferably, REAC2 is done at a pressure from atmospheric pressure to 20 bar, more preferably from atmospheric pressure to 10 bar, even more preferably from atmospheric pressure to 5 bar, especially, REAC2 is done at atmospheric pressure.
Preferably, the reaction time TIME2 of REAC2 is from 1 h to 12 h, more preferably from 1 h to 6 h.

REAC2 can be performed under an atmosphere of air or under an atmosphere of an inert gas, such as nitrogen or such as a noble gas, such as argon.

REAC2 can be done in a solvent SOLV2, SOLV2 is preferably dichloromethane, dichlorethylene, chlorbenzene, toluene, MTBE, C₂₋₄ alkyl acetate, triethylamine, diisopropyl amine;
more preferably dichloromethane.
Preferably, REAC2 is done in SOLV2.

Preferably, after REAC2 a quencher such as water is added to the reaction mixture.
Preferably, the amount of water, that is added, is from 0.1 to 20 times, more preferably from 0.1 to 10 times, of the weight of the reaction mixture.

After REAC2, compound of formula (III) can be isolated and purified by conventional methods, which are known to those skilled in the art. These conventional methods include extraction, crystallization, chromatography, or any combination of these methods of purification.

Preferably, compound of formula (II) is prepared by a reaction REAC 1 of compound of formula (I) 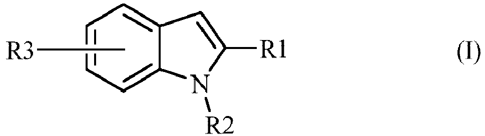 with compound SULFON;
SULFON is selected from the group consisting of compound of formula (SULPYR), chloro sulfonic acid and oleum; 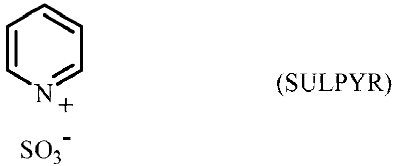 with R1, R2 and R3 as defined herein, also with all their embodiments.

Preferably SULFON is compound of formula (SULPYR).

Preferably, the molar amount of SULFON is from 1 to 5 times, more preferably from 1 to 3 times, even more preferably from 1 to 2 times, of the molar amount of compound of formula (I).

Preferably, the reaction temperature TEMP1 of REAC1 is from 0 to 100°C, more preferably from 10 to 80°C, even more preferably from 20 to 80°C, especially from 30 to 60°C.
Preferably, the pressure PRESS 1 of REAC1 is adjusted according to the vapor pressure of the reaction mixture at the chosen TEMP1 of REAC1; but PRESS1 can also be adjusted to a higher pressure than the vapor pressure of the reaction mixture at the chosen TEMP1. A PRESS1 higher than the vapor pressure of the reaction mixture at the chosen TEMP1 can be adjusted for example by applying inert gas such a nitrogen or argon to the reaction vessel;
more preferably, REAC 1 is done at a pressure from atmospheric pressure to 20 bar, more preferably from atmospheric pressure to 10 bar, even more preferably from atmospheric pressure to 5 bar, especially, REAC 1 is done at atmospheric pressure.
Preferably, the reaction time TIME1 of REAC1 is from 1 h to 12 h, more preferably from 1 h to 8 h.

REAC1 can be performed under an atmosphere of air or under an atmosphere of an inert gas, such as nitrogen or such as a noble gas, such as argon.

REAC1 can be done in a solvent SOLV1, SOLV1 is preferably dichloromethane, chlorbenzene, dichlorethane, pyrdine, picoline, chinoline, or a mixture thereof; preferably dichloromethane.
Preferably, REAC1 is done in SOLV 1.

Preferably, SOLV1 and SOLV2 are identical.

After REAC1, compound of formula (II) can be isolated and purified by conventional methods, which are known to those skilled in the art. These conventional methods include extraction, crystallization, chromatography, or any combination of these methods of purification.

Preferably, compound of formula (II) is not isolated.
Preferably, compound of formula (II) is used in REAC2 in form of the reaction mixture obtained from REAC1.

Compound of formula (I) and SULFON are known compounds and can be prepared by known methods.

Another of the invention is the use of compound of formula (V) for the preparation of compound of formula (VI); 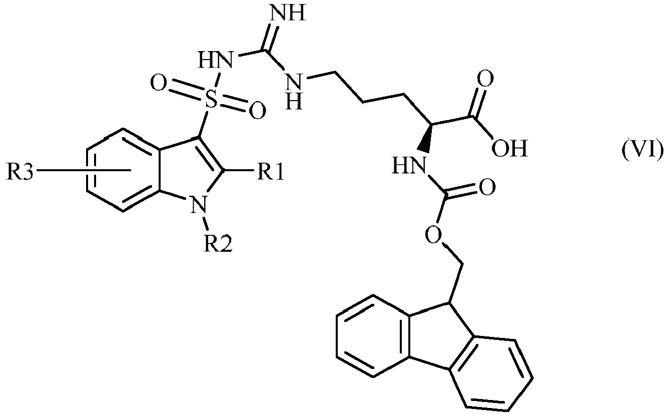 with R1, R2 and R3 as defined herein, also with all their embodiments.

Another of the invention is the method for the preparation of compound of formula (VI) by a reaction REAC5 of compound of formula (V) with a compound FMOCACT; FMOCACT is an activated form of Fmoc, preferably such as Fmoc-Cl or compound of formula (Fmoc-OSu).

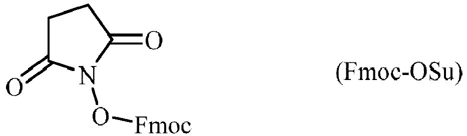

Preferably, compound of formula (V) has been prepared by REAC4, with REAC4 as described herein.

Preferably FMOCACT is compound of formula (Fmoc-OSu).

Preferably, the molar amount of FMOCACT is from 1 to 5 times, more preferably from 1 to 3 times, even more preferably from 1 to 2 times, of the molar amount of compound of formula (V).

Preferably, the reaction temperature TEMP5 of REAC5 is from 0 to 100°C, more preferably from 10 to 80°C, even more preferably from 10 to 60°C, especially from 10 to 40°C.

Preferably, the pressure PRESS5 of REAC5 is adjusted according to the vapor pressure of the reaction mixture at the chosen TEMP5 of REAC5; but PRESS5 can also be adjusted to a higher pressure than the vapor pressure of the reaction mixture at the chosen TEMP5. A PRESS5 higher than the vapor pressure of the reaction mixture at the chosen TEMP5 can be adjusted for example by applying inert gas such a nitrogen or argon to the reaction vessel;
more preferably, REAC5 is done at a pressure from atmospheric pressure to 20 bar, more preferably from atmospheric pressure to 10 bar, even more preferably from atmospheric pressure to 5 bar, especially, REAC5 is done at atmospheric pressure.
Preferably, the reaction time TIME5 of REAC5 is from 1 h to 12 h, more preferably from 1 h to 8 h, even more preferably from 1 h to 6 h.

REAC5 can be performed under an atmosphere of air or under an atmosphere of an inert gas, such as nitrogen or such as a noble gas, such as argon.

REAC5 can be done in a solvent SOLV5, SOLV5 is preferably water, acetonitrile, dichlorethane, methylenchloride, toluene, MTBE, DMAA, tetramethylurea, or a mixture thereof;
more preferably water, acetonitrile or a mixture thereof.
Preferably, REAC5 is done in SOLV5.

After REAC5, compound of formula (VI) can be isolated and purified by conventional methods, which are known to those skilled in the art. These conventional methods include extraction, crystallization, chromatography, or any combination of these methods of purification.

A preferred embodiment of compound of formula (V) is compound of formula (5).

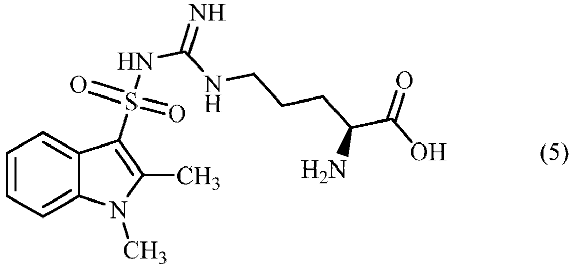

A preferred embodiment of compound of formula (IV) is compound of formula (4).

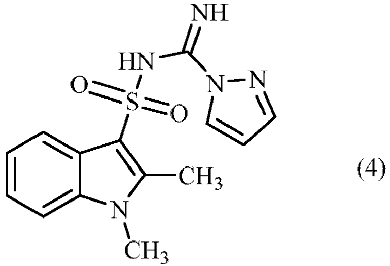

A preferred embodiment of compound of formula (III) is compound of formula (3).

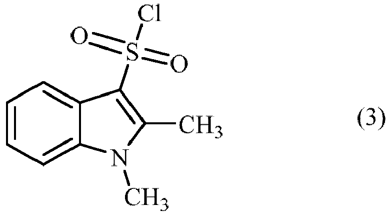

A preferred embodiment of compound of formula (II) is compound of formula (2).

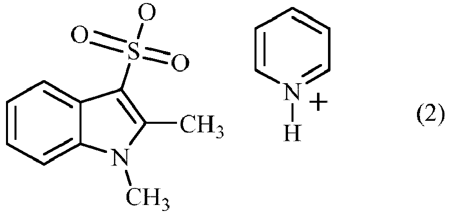

A preferred embodiment of compound of formula (I) is compound of formula (1).

A preferred embodiment of compound of formula (VI) is compound of formula (6).

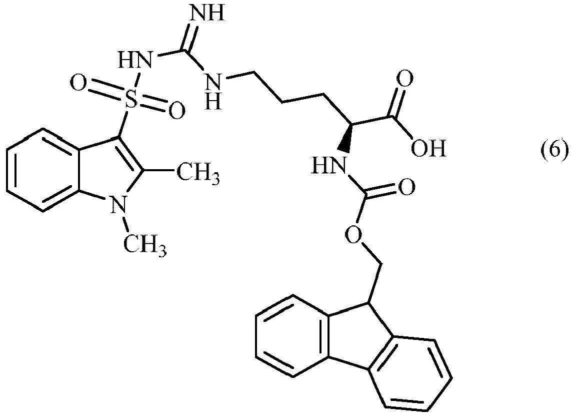

### Examples

### Methods:

| **HPLC for Examples 1,2 and 3:** | | | |
|---|---|---|---|
| Mobile phase A: | 0.045% TFA in water | | |
| Mobile phase B: | 0.036%TFA in Acetonitrile | | |
| Column | ZORBAX Eclipse™ XDB-C₈ 5 micrometer; 4.6×150 mm; S.N. USRK041807 | | |
| Flow | 1 mL/min | | |
| Injection | 2 microL | | |
| UV | 254 nm | | |

| Gradient | Time [min] | A [%] | B [%] |
|---|---|---|---|
| | 0 | 95 | 5 |
| | 15 | 0 | 100 |
| | 15.1 | 95 | 5 |
| | 20 | 95 | 5 |

| **HPLC for Example 5 - purity:** | | | |
|---|---|---|---|
| Mobile phase A: | 0.1 % TFA in water | | |
| Mobile phase B: | 0.1 %TFA in Acetonitrile | | |
| Column | Xterra RP C18; 5 micrometer; 4.6 x 150 mm | | |
| Flow | 1 mL/min | | |
| Injection | 2 microL | | |
| UV | 254 nm | | |

| Gradient | Time [min] | A [%] | B [%] |
|---|---|---|---|
| | 0 | 95 | 5 |
| | 20 | 5 | 95 |
| | 25 | 5 | 95 |
| | 25.1 | 95 | 5 |

| **HPLC for Example 5 - Chiral purity:** | | | |
|---|---|---|---|
| Mobile phase A: | Ammonium acetate buffer 100 mM, pH=6.0 (7.71 gr Ammonium Acetate are dissolved in 1000 ml water, the solution is adjusted to pH=6.0 with glacial acetic acid) | | |
| Mobile phase B: | Methanol | | |
| Column | ChiralPak QN-AX; 150 x 4.6 mm; 5 micrometer | | |
| Flow | 1 mL/min | | |
| Injection | 10 microL | | |
| concentration | 1 mg/mL | | |
| UV | 254 nm | | |

| Isocratic | A [%] | B [%] | |
|---|---|---|---|
| | 25 | 75 | |

### Example 1, Synthesis of MIS-OH.Py, compound of formula (2)

1,2-Dimethylindole (1.00 kg, 6.88 mol, 1.00 eq), sulphur trioxide pyridine complex (1.42 kg, 8.88 mol, 1.29 eq) and dichloromethane (6.7 kg) were mixed. The mixture was heated to 40°C and kept for 4 h at 40°C. The conversion reached 99.5% as determined by HPLC. If the value is not reached, then 0.03 eq of sulphur trioxide pyridine complex can be added and stirring can be continued at 40°C for 1 h. The reaction mixture was cooled down to 20 to 30°C and was used for next step (based on the assumption of 100% yield, containing 2.09 kg of MIS-OH.Py, 6.88 mol).
The structure was confirmed by ¹H NMR.

### Example 2, Synthesis of MIS-Cl, compound of formula (3)

To the reaction mixture containing MIS-OH.Py in dichloromethane, prepared according to example 1, were added 12.7 kg of dichloromethane. DMF (50 g, 0.68 mol, 0.1 eq) was added. The reaction mixture was cooled down to 0 to 5°C. Oxalyl chloride (1.19 kg, 9.34 mol, 1.36 eq) was added dropwise in 2 h while keeping the reaction temperature at 0 to 5°C. Then the reaction mixture was kept at 0 to 5°C for 1 h. The conversion reached 99.5% by HPLC analysis.

The reaction mixture was quenched by addition of water (2.2 kg) at -5 to 5°C while stirring. Two phases separated, the water phase was discarded. The organic phase was washed with water (twice with 2.0 kg) at 0 to 5°C. Dichloromethane (2.7 kg) was charged and the resulting mixture solution was heated to 25 to 30°C. Anhydrous MgSO₄ (700 g) was charged and the reaction mixture was stirred for 0.5 h. MgSO₄ was removed by filtration and the MgSO₄ filter cake was washed with dichloromethane(650 g), the filtrates were combined. The combined filtrates were concentrated to about 2.3 kg under 250 to 300 mbar pressure and at 15 to 20°C. MIS-Cl was obtained by filtration and the filter cake was washed with a mixture of dichloromethane and hexane (v/v = 1/1,3 L). The collected pink MIS-Cl was dried at 15 to 20°C for 15 h. 1.4 kg of MIS-Cl (84% yield, this is the combined yield of example 1 and example 2) was obtained with 99% HPLC purity.
The yield of example 1 and example 2 each was ca. 92%.
The structure was confirmed by ¹H NMR and ¹³C NMR.

### Example 3, Synthesis of compound of formula (4)

Pyrazole-1-carboxamidine hydrochloride (0.85 kg, 5.80 mol, 1.0 eq), MeCN(5.10 kg) and DIPEA(1.92 kg,14.8 mol, 2.6 eq) were mixed. The temperature of mixture is adjusted to 18 to 22°C. A solution of MeCN (1.50 kg) and MIS-Cl (prepared according to example 2, 1.41 kg, 5.80 mol, 1.0 eq) was added dropwise in 0.5 to 1 h. The mixture was stirred at 18 to 22°C for 16 h. The mixture was filtered and the obtained filter cake was washed with H₂O (5.8 kg), followed by washing with MeCN (0.5 kg). The filter cake was dried at 38°C and 5 mbar for 16 h. Compound of formula (4) (1.2 kg) was obtained as white solid with 98% HPLC purity, 65% isolation yield.
The structure was confirmed by ¹H NMR and ¹³C NMR.

### Example 4, Synthesis of L-Arg(MIS)-OH, compound of formula (5)

H₂O (1.23 kg) and L-Orn.HCl (1.04 kg, 6.17 mol, 1.8 eq) were mixed, a solution of LiOH.H₂O (0.41 kg, 9.95 mol, 2.9 eq) in H₂O (3.50 kg) was added dropwise in 0.5 h at below 30°C. The mixture was heated to 50°C and the pH was adjusted to 10.8 with a 3M aqueous solution of LiOH (about 1 kg). MeCN (1.58 kg) was added. Compound of formula (4) (prepared according to example 4, 1.1 kg, 3.43 mol, 1.0 eq) was added, then MeCN (1.0 kg) was added. The reaction mixture was stirred for 16 h at 60°C. A solution of CuCl2.H₂O (0.57 kg, 3.77mol, 1.1 eq) in water (1.93 kg) was added in 3 h at 60°C. The mixture, that was a solution a solution before the addition of the CuCl₂, became a suspension during the addition of the CuCl₂. The suspension was stirred for 2 h. The solid was collected by filtration at 60°C and was washed with H₂O (3 times with 5 kg, 60°C), followed by washing with EtOH (4 kg, 60°C).
The wet solid was mixed with EDTA-2Na.2H₂O (1.15 kg, 3.08mol, 0.9 eq) and H₂O (7.7 kg). The mixture was heated to 65°C and stirred for 3 h. The mixture was cooled to 0°C in 3 h. The mixture was filtered and the filter cake was washed with H₂O (3 times witzh 3 kg of 0°C). Drying was done at 42°C under a pressure below 15 mbar for 16 h. 1.02 kg of L-Arg(MIS)-OH were obtained, yield was 78%.
The structure was confirmed by ¹H NMR and ¹³C NMR.

**Example 5, Synthesis of Fmoc-Arg(MIS)-OH, compound of formula (6)** L-Arg(MIS)-OH (prepared according to example 4, 1.0 kg, 2.60mol,1.0 eq) was mixed with a solution of Na₂CO₃ (0.29 kg, 2.74 mol,1.05 eq) in H₂O(5.3 kg), MeCN (7.1 kg) were charged. The mixture was stirred at 20°C for 30 min, L-Arg(MIS)-OH was dissolved completely. A solution of Fmoc-OSu (0.87 kg, 2.60 mol, 1.0 eq) in MeCN(0.8 kg) was added dropwise in 15 min. The reaction mixture was stirred for another 3, the content of of L-Arg(MIS)-OH was lower than 0.2%. as determined by HPLC.
H₂O (5.3 kg) was added to the reaction mixture, the resulting mixture was washed with diisopropylethyl ether (3 times with 10 L). Ethyl acetate (36 kg) was added, the mixture was cooled to 0 to 5°C, the pH was adjusted to 3.8 to 4.0 with a 30 wt-% citric aqueous solution (about 3.3 kg) at 0 to 5°C. Two phases had formed and were separated, the organic phase was washed with water (3 times with 8 kg), the organic phase was concentrated to a 4 L suspension under 50 to 300 mbar at a jacket temperature of the reaction vessel of below 30°C. MeOH (5.9 kg) was charged and and the concentration was continued to 4 L under 50 to 300 mbar at a jacket temperature of below 30°C. The product was collected by filtration and was dried under vacuum to give 1.2 kg product, yield: 80.1 %.
The structure was confirmed by ¹H NMR and ¹³C NMR.
Purity was 99.5%.
Chiral purity ee was 99.8%.

## Claims

1. A method for the preparation of compound of formula (V) 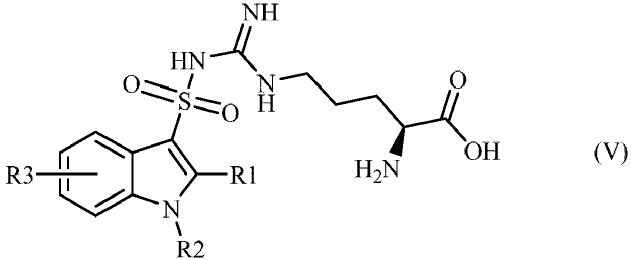 by a reaction REAC4, wherein compound of formula (IV) 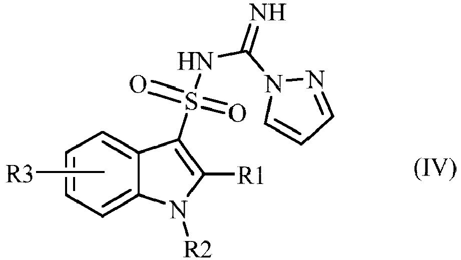 is reacted with L-ornithine;
R1 is H, C₁₋₆ alkyl, C₁₋₆ alkoxy or C₁₋₆ alkylthio;
R2 is C₁₋₆ alkyl, C₁₋₆ alkoxy or C₁₋₆ alkylthio;
or R1 and R2 together form a moiety of formula -(CH₂)n-,
n 3, 4 or 5;
R3 is H, halogen, C₁₋₆ alkyl, C₁₋₆ alkoxy, C₁₋₆ alkylthio, phenyl or benzyl.

2. Method according to claim 1, wherein
R1 is C₁₋₄ alkyl, C₁₋₄ alkoxy or C₁₋₄ alkylthio;
R2 is C₁₋₄ alkyl, C₁₋₄ alkoxy or C₁₋₄ alkylthio;
R3 is H, halogen, C₁₋₄ alkyl, C₁₋₄ alkoxy, C₁₋₄ alkylthio, phenyl or benzyl.

3. Method according to claim 1 or 2, wherein
compound of formula (IV) is prepared by a reaction REAC3 of compound of formula (III) with compound of formula (PYCAAM); 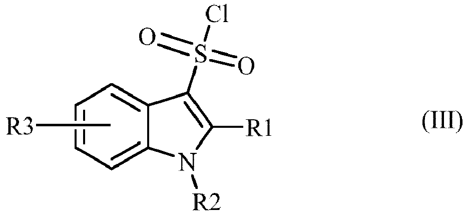 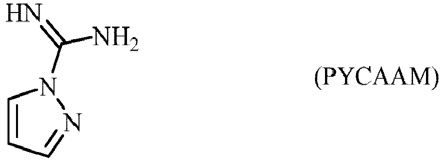 with R1, R2 and R3 as defined in claim 1.

4. Method according to claim 3, wherein
compound of formula (III) is prepared by a reaction REAC2 of compound of formula (II) with a halogenating agent HALAG2; 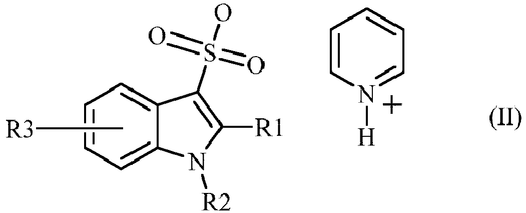 with R1, R2 and R3 as defined in claim 1.

5. Method according to claim 4, wherein
HALAG2 is selected from the group consisting of oxalyl chloride, thionylchloride, phosgene, diphosgene, triphosgene, POCl₃, PCl₅, Cl₂ in the presence of triphenylphoshine, cyanuric chloride, and methansulfonic acid chloride.

6. Method according to claim 4 or 5, wherein
compound of formula (II) is prepared by a reaction REAC1 of compound of formula (I) 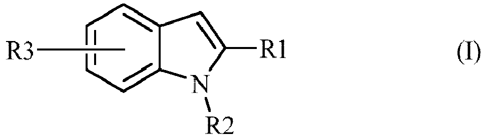 with compound SULFON, SULFON is selected from the group consisting of compound of formula (SULPYR), chloro sulfonic acid and oleum; 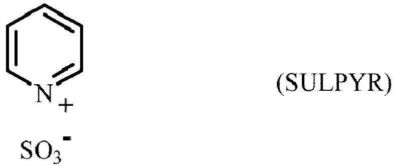 with R1, R2 and R3 as defined in claim 1.

7. Method for the preparation of compound of formula (VI); 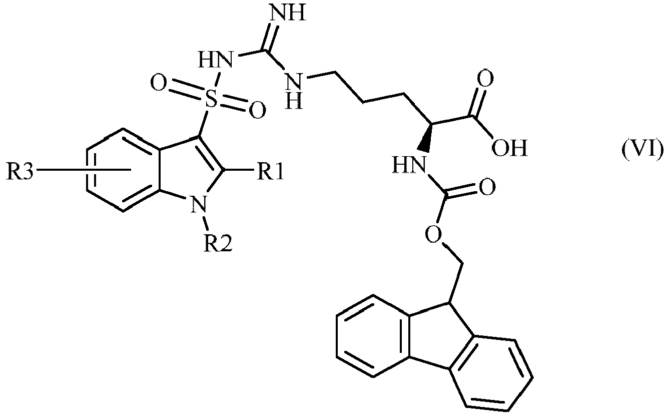 with R1, R2 and R3 as defined in claim 1;
with compound of formula (V) as defined in claim 1;
by a reaction REAC5 of compound of formula (V) with compound FMOCACT;
FMOCACT is an activated form of Fmoc;
and wherein compound of formula (V) has been prepared by REAC4, with REAC4 as defined in claim 1.

8. Method according to claim 7, wherein FMOCACT is Fmoc-Cl or compound of formula (Fmoc-OSu).
